# EUROPEAN PATENT APPLICATION

(11) **EP 4 306 638 A1**
(43) Date of publication of application: **17.01.2024**
(21) Application number: 22887491.3
(22) Date of filing: 20.10.2022
(51) Int. Cl.: C12N 9/04, C12N 15/70, C12P 33/00

(54) **VARIANT OF RUMINOCOCCUS GNAVUS STRAIN-DERIVED 7-BETA-HSDH, IMPROVING CONVERSION RATE FROM 7-KETO-LCA TO URSODEOXYCHOLIC ACID, AND METHOD FOR PRODUCING URSODEOXYCHOLIC ACID BY USING SAME**

(30) Priority: 25.10.2021 KR 20210142625
(71) Applicant: Ace Biopharm Co., Ltd., Seoul 06784 (KR)
(72) Inventor: MIN, Byeong Gyu, Yongin-si Gyeonggi-do 16806 (KR); JANG, Hyung Wook, Sejong 30098 (KR); PARK, Jae Bum, Daejeon 34116 (KR); KIM, So Young, Daejeon 34066 (KR); SOHN, Min Jeong, Daejeon 35206 (KR)
(74) Representative: Vossius & Partner Patentanwälte Rechtsanwälte mbB
(86) International application number: PCT/KR2022/016088
(87) International publication number: WO 2023/075295

(57) **Abstract**

Provided is a variant of *RUMINOCOCCUS GNAVUS* strain-derived 7 beta-hydroxysteroid (7-beta-HSDH) to improve the conversion rate from 7-keto lithocholic acid (7-Keto-LCA) to ursodeoxycholic acid (UDCA), and a method of producing UDCA using the same. The conversion efficiency of 7-Keto-LCA to UDCA is improved through the preparation of variants via enzyme modification and the development of recombinant protein production, which is expected to be widely utilized to improve UDCA biosynthesis and biosynthetic efficiency.

## Description

### Technical Field

The present disclosure relates to a variant of *RUMINOCOCCUS GNAVUS* strain-derived 7 beta-hydroxysteroid dehydrogenase (7-beta-HSDH) to improve the conversion rate from 7-keto lithocholic acid (7-Keto-LCA) to *ursodeoxycholic acid* (UDCA) and a method of producing UDCA using the same.

### Background Art

Bile acids are a major constituent of bile and are biomolecules required for the digestion and absorption of fats, fatty acids and hydrophobic vitamins. Bile acids are a stable substance that is not easily metabolized, and various hydroxy groups that cause differences in biochemical function and solubility are arranged at various carbon positions in the central cholesterol ring. From among the various types of bile acids, ursodeoxycholic acid (UDCA) is one of the bile acids found only in small amounts. UDCA has recently been found to have a therapeutic function in the dissolution of cholesterol-including gallstones. This compound is produced industrially in tonne quantities in chemical or enzymatic steps. An important precursor for the synthesis of UDCA is 7-keto lithocholic acid (7-Keto-LCA), which can be converted to UDCA by reacting 7-Keto-LCA with n-butanol in the presence of sodium as a catalyst. In the case of methods using enzymes, the conversion into UDCA may be performed by using an enzymatic catalysis catalyzed by 7 beta-hydroxysteroid dehydrogenase (7-beta-HSDH), which can stereoselectively reduce the 7-keto group. This step uses an enzymatic catalysis catalyzed by 7-beta-HSDH and requires the coenzyme NAD(P)H in the reaction. However, in the case of the conversion reaction from 7-Keto-LCA to UDCA using 7-beta-HSDH of the conventional *RUMINOCOCCUS GNAVUS* strain, when 30 g/L or more of 7-Keto-LCA is present as a substrate, the conversion ability is reduced due to inhibition by the substrate concentration.

Therefore, there is a need for research on mitigating the effect of decreasing 7-beta-HSDH enzyme activity due to an increase in substrate concentration by changing the amino acid sequence of the substrate binding site, or mass production of the improved enzyme using recombinant *Escherichia coli,* and research in this field is still lacking.

### Detailed Description of the disclosure

### Technical Problem

One aspect is to provide a 7 beta-hydroxysteroid dehydrogenase (7-beta-HSDH) variant including an amino acid sequence mutation of 7-beta-HSDH derived from *RUMINOCOCCUS GNAVUS* strain.

Another aspect is to provide a recombinant vector including the variant.

Another aspect is to provide a cell that is transformed with the recombinant vector.

Another aspect is to provide a method of producing ursodeoxycholic acid (UDCA) for increasing the conversion rate of 7-keto lithocholic acid (7-Keto-LCA) to UDCA, the method including converting 7-Keto-LCA to UDCA by 7-beta-HSDH of which the substrate binding site is mutated.

Another aspect is to provide a method of increasing the conversion rate of 7-Keto-LCA to UDCA, the method including converting 7-Keto-LCA to UDCA by 7-beta-HSDH of which the substrate binding site is mutated.

### Technical Solution to Problem

One aspect is to provide a 7 beta-hydroxysteroid dehydrogenase (7-beta-HSDH) variant including an amino acid sequence mutation of *RUMINOCOCCUS GNAVUS* strain-derived 7-beta-HSDH,
wherein the amino acid sequence mutation includes one or more mutations selected from the group consisting of 1) M207X₁, 2) T210X₂, and 3) Q212X₃, X₁ represents an amino acid other than methionine (M), and X₂ represents an amino acid other than threonine (T), and X₃ represents an amino acid other than glutamine (Q).

In an embodiment, the 7-beta-HSDH variant additionally may have 4) D252X₄ mutation, and X₄ may be an amino acid other than aspartic acid (D).

The term "7-beta-HSDH" used herein refers to the stereospecific reduction (hydrogenation) of the corresponding 7-beta-HSDH of 7-ketosteroids, or the corresponding 7-beta-HSDH of ketosteroid including a keto group on the 7^{th}-position and one or more additional keto groups on the steroid structure.

The term "amino acid" used herein may be selected from alanine (A; A), cysteine (C; Cys), aspartic acid (D; Asp), glutamic acid (E; Glu), phenylalanine (F; Phe), glycine (G; G), histidine (H; His), isoleucine (I; Ile), lysine (K; Lys), leucine (L; Leu), methionine (M; Met), asparagine (N; Asn), proline (P; Pro), glutamine (Q; Gln), arginine (R; Arg), serine (S; Ser), threonine (T; Thr), valine (V; Val), tryptophan (W; Trp), or tyrosine (Y; Tyr). For example, the amino acid may be valine (V; Val), isoleucine (I; Ile), leucine (L; Leu), or glutamic acid (E; Glu), but is not limited thereto.

The term "amino acid sequence mutation" used herein refers to a mutation in one or more amino acid sequences, and may be one selected from the group consisting of M207X₁, T210X₂, Q212X₃ and D252X₄, each representing one mutation, wherein X₁ to X₄ may be selected from valine (V; Val), isoleucine (I; Ile), leucine (L; Leu), or glutamic acid (E; Glu), specifically, X₁ may be valine (V; Val), X₂ may be isoleucine (I; Ile), X₃ may be leucine (L; Leu), and X₄ may be glutamic acid (E; Glu).

In addition, in the case of having multiple amino acid sequence variations, the multiple amino acid sequence variations may be selected from (M207X₁ and Q212X₃), (M207X₁ and D252X₄), (T210X₂ and Q212X₃), (T210X₂ and D252X₄), (M207X₁, Q212X₃, and D252X₄), (M207X₁, T210X₂, and Q212X₃), (M207X₁, T210X₂, and D252X₄), (T210X₂, Q212X₃, and D252X₄), or (M207X₁, T210X₂, Q212X₃, and D252X₄), whereinXi to X₄ may be selected from valine (V; Val), isoleucine (I; Ile), leucine (L; Leu) , or glutamic acid (E; Glu), and specifically, X₁ may be valine (V; Val), X₂ may be isoleucine (I; Ile), X₃ may be leucine (L; Leu), and X₄ may be glutamic acid (E; Glu).

In an embodiment, the 7-beta-HSDH variant may additionally have at least one mutation in a sequence motif corresponding to an amino acid sequence having at least 80% sequence homology with SEQ ID NO: 1, wherein the sequence motif 207 to 212 of SEQ ID NO: 1 in the mutation may be VMKIAL, but is not limited thereto.

The term "variant" used herein refers to a polypeptide in which one or more amino acids have been conservatively substituted and/or modified such that the amino acid sequence of the variant differs from the amino acid sequence thereof before mutation but the functions or properties are retained. Such variants can generally be identified by modifying one or more amino acids in the amino acid sequence of the polypeptide and evaluating the properties of the modified polypeptide. That is, the ability of the variant may be increased, unchanged, or reduced, compared to the polypeptide before the mutation. In addition, some variants may include variants in which one or more portions such as an N-terminal leader sequence or a transmembrane domain are removed. Other variants may include variants in which a portion is removed from the N- and/or C-terminus of the mature protein. The term "variant" may be used interchangeably with the terms of modified, modification, modified polypeptide, mutated protein, mutation, and variant, and the like, so long as the term is used having the meaning of mutation. For purposes of the present application, the variant may be the polypeptide as set forth in SEQ ID NO: 1 in which threonine, the 210th amino acid of the amino acid sequence of SEQ ID NO: 3, is substituted with isoleucine, and glutamine, the 212th amino acid thereof, is substituted with leucine.

The term "stereo selectivity" used herein refers to a property that has a single three-dimensional structure and is selectively produced during a chemical reaction.

In an embodiment, the 7-beta-HSDH variant may additionally have at least one mutation in a sequence motif corresponding to an amino acid sequence having at least 80% sequence identity with SEQ ID NO: 1. The variant may consist of an amino acid sequence having at least 80%, at least 90%, at least 95%, at least 96%, at least 97%, at least 98%, or at least 99% sequence homology with SEQ ID NO: 1, and the mutation may be such that the 207 to 212 sequence motif of SEQ ID NO: 1 is VMKIAL.

The term "sequence motif" used herein refers to, as a component of the sequence, the sequence 207 to 212 of SEQ ID NO: 1, which is the substrate binding site of the *Ruminococcus gnavu (R. gnavus)*-derived 7-beta-HSDH amino acid sequence.

In another embodiment, the *RUMINOCOCCUS GNAVUS* strain-derived 7-beta-HSDH recognizes 7-Keto-LCA as a substrate and induces catalysis of stereospecific enzymatic reduction at the 7th carbon.

The term "stereospecificity" used herein refers to a case in which in a reaction, one particular stereoisomer forms one particular stereoisomer (or pair of enantiomers) product, in which the stereochemical result generated in a given reactant can be clearly specified.

The term "enzymatic reduction" used herein refers to a method using a microbial-derived reductase. According to the enzymatic reduction method, a reductase gene is separated from a novel microorganism isolated from the nature system or a microorganism known in a paper or patent, and the separated gene is transplanted into a plasmid and transformed in *Escherichia coli.* (*E. col*), the resulting *E. coli* is cultured, and then the cells are crushed and centrifuged, and the resulting organism can be used in the reaction as is, or the supernatant recovered by crushing and centrifuging the *E. coli* can be used in the reaction. The catalysis of enzymatic reduction may be induced using a *RUMINOCOCCUS GNAVUS* strain, but is not limited thereto.

Another aspect provides a recombinant vector containing the gene of the 7-beta-HSDH variant.

In an embodiment, the variant contained in the recombinant vector may consist of an amino acid sequence having a sequence homology of at least 80%, for example, at least 90%, at least 95%, at least 96%, at least 97%, at least 98%, and at least 99% to SEQ ID NO: 1.

In an embodiment, the variant may consist of the nucleotide sequence of SEQ ID NO: 2.

The term "gene" as used herein refers to a structural unit that determines genetic information, and includes a structural gene having information that determines the amino acid sequence of a protein or the base sequence of a functional RNA (tRNA, rRNA, etc.), and/or a regulatory gene (e.g., promoter, repressor, operator, etc.) that controls the expression of the structural gene, and is understood to refer to the single-stranded side containing the nucleotide sequence that is transcribed to produce the product of the gene.

The term "vector" as used herein refers to a nucleic acid molecule capable of transporting another nucleic acid to which the vector is linked. Vectors include: nucleic acid molecules that are single-stranded, double-stranded, or partially double-stranded; nucleic acid molecules that include one or more free ends, nucleic acid molecules that lack free ends (e.g., circular); nucleic acid molecules that include DNA, RNA, or DNA and RNA; and various other polynucleotides known in the art.

The term "recombinant vector" as used herein refers to an expression vector for a target polypeptide that, when operably linked to a gene encoding the target polypeptide (nucleic acid) to be expressed, is capable of expressing the target polypeptide with high efficiency in a suitable host cell, and the recombinant vector is expressible in the host cell. The host cell may be a prokaryotic cell, and depending on the type of host cell, expression control sequences such as a promoter, terminator, and enhancer, sequences for membrane targeting or secretion, etc. may be appropriately selected and can be combined in various ways according to the purpose.

In an embodiment, the recombinant vector may include a Tac promoter and a kanamycin resistance gene.

The term "promoter," as used herein, refers to a nucleotide sequence that acts to determine when, where, and to what extent a gene is expressed, i.e., a regulatory region that has the function of controlling the expression of a gene. The promoter is a synthetic DNA promoter generated from a combination of promoters from the trp and lac operons. Generally, the Tac promoter used for the production of proteins in *E. coli* can be used.

The term "kanamycin resistance gene," as used herein, refers to a gene exhibiting drug resistance that allows a recombinant microorganism to survive when exposed to an antibiotic.

Another aspect is to provide a cell that is transformed with the recombinant vector.

The term "transformation," as used herein, may refer to the alteration of the genetic characteristics of an organism by externally provided DNA. That is, the transformation refers to a case where, when DNA, a type of nucleic acid derived from cells of one strain of an organism, is introduced into a living cell of a different strain, the DNA enters the cell and causes a change in genotype.

In an embodiment, the transformed cells may be obtained by introducing a recombinant vector into *E. coli* to perform the transformation, and the transformed cells may be one deposited under Accession No. KCTC 14715 BP.

In an embodiment, the *E. coli* may be cultured in a fermentation medium including LB medium at 30 °C to 40 °C for 4 hours to 48 hours, wherein the culture may be performed under aerobic conditions using a baffled flask, but embodiments are not limited thereto.

The term "expression" used herein refers to the introduction of a gene of foreign origin into a strain of *E. coli* to artificially express an enzyme that the strain is unable to express on its own, and the term "overexpression" used herein refers to a case where the *E. coli* strain has the gene encoding the corresponding enzyme and is able to express the same on its own, but has artificially increased the expression of the enzyme and overexpressed the gene in order to increase the expression thereof for the purpose of mass production.

The term "primer" used herein is a nucleic acid sequence having a short free 3' hydroxyl group, capable of forming a base pair with a complementary nucleic acid template, and a short nucleic acid sequence acting as a starting point for strand copying of a nucleic acid template. A primer can initiate DNA synthesis in the presence of a reagent for polymerization (i.e., DNA polymerase or reverse transcriptase) in an appropriate buffer and temperature and different four nucleotide triphosphates.

When designing the primers, various restrictions are followed: the A, G, C, and T content ratio of the primers, prevention of formation of primer dimer, and prohibition of repeating the same nucleotide sequence three or more times. Besides, regarding the conditions of a single-step PCR reaction, the amount of template DNA, concentration of primers, concentration of dNTPs, concentration of Mg²⁺, reaction temperature, and reaction time should be appropriate.

The primers can be incorporated with additional features that do not change basic properties. That is, nucleic acid sequences can be modified using a number of means known in the art. Examples of such modifications are methylation, capping, substitution of nucleotides with one or more homologues, and transformation of nucleotides into uncharged linkages such as phosphonates, phosphotriesters, hosphoroamidates or carbamates, or charged linkages such as phosphorothioates or phosphorodithioates. Nucleic acids may also have an additional covalently bonded residue of one or more of: proteins such as nucleases, toxins, antibodies, signal peptides, or poly-L-lysine; intercalating agents such as acridine or psoralen; chelating agents and alkylating agents such as metals, radioactive metals, or iron oxidizing metals, etc.

In an embodiment, the transformed cells may be those transformed by introducing, to express an enzyme (SEQ ID NO: 1) in which four amino acids of the 7-beta-HSDH enzyme have been altered, an expression vector into which the gene is inserted into *E. coli* MG1655, and as for the recombinant *E. coli* ABP-5 (Accession No. KCTC 14715 BP), ABP-5, which is 7-beta-HSDH enzyme variant improved by a protein expression process using a corresponding strain derived from *R. gnavus* strain, may be ensured.

Another aspect is to provide a method of producing ursodeoxycholic acid (UDCA) for increasing the conversion rate of 7-Keto-LCA to UDCA, the method including converting 7-Keto-LCA to UDCA by 7-beta-HSDH of which the substrate binding site is mutated.

Another aspect is to provide a method of increasing the conversion rate of 7-Keto-LCA to UDCA, the method including converting 7-Keto-LCA to UDCA by 7-beta-HSDH of which the substrate binding site is mutated.

In an embodiment, the substrate binding site of the enzyme may be a sequence motif 200 to 215 of the amino acid sequence of 7-beta-HSDH, but is not limited thereto.

In an embodiment, the mutation may include one selected from the group consisting of 1) M207X₁, 2) T210X₂, and 3) Q212X₃, X₁ represents an amino acid other than methionine (M), and X₂ represents an amino acid other than threonine (T), and X₃ represents an amino acid other than glutamine (Q). However, embodiments are not limited thereto.

The term "7-Keto-LCA" used herein refers to an intermediate in the synthesis of ursodeoxycholic acid (UDCA), which is a secondary bile acid by human intestinal bacteria, and an intermediate formed from chenodeoxycholic acid.

The term "conversion rate" used herein refers to the percentage of an original specific substance (raw material) that is converted into another substance by a chemical reaction based on the number of moles of the original specific substance (raw material).

### Advantageous Effects of Disclosure

According to the present disclosure, the conversion efficiency of 7-Keto-LCA to UDCA is increased through the preparation of variants via enzyme modification and the development of recombinant protein production, and thus it is expected to be widely utilized for biosynthesis and improvement of biosynthetic efficiency of UDCA.

### Brief Description of Drawings

FIG. 1 shows a result of comparing *Ruminococcus gnavu (R. gnavus)*-derived 7 beta-hydroxy steroid dehydrogenase (7-beta-HSDH) (SEQ ID NO: 3) and the amino acid sequence of ABP-5 modified according to the present disclosure (SEQ ID NO: 1).
FIG. 2 shows the pCDFDuet-1-Tac-Kan vector (Tac promoter, kanamycin resistance) used to express ABP-5, which is an improved 7-beta-HSDH enzyme.
FIG. 3 shows the results of SDS-PAGE analysis of the expressions of *R. gnavus-*derived 7-beta-HSDH and modified *R. gnavus* 7-beta-HSDH (ABP-5) enzyme, using *Escherichia coli.* (A: *R. gnavus* 7-beta-HSDH enzyme (29.32 kDa), B: ABP-5 (29.3 kDa))
FIG. 4 shows a schematic diagram of conversion of 7-Keto-LCA to UDCA using 7-beta-HSDH enzyme including a coenzyme regeneration system.
FIG. 5 shows the results of comparing the UDCA conversion rate of each enzyme at different 7-Keto-LCA concentrations. (○: *R. gnavus* 7-beta-HSDH ■: ABP-5)
FIG. 6 shows the results of comparing the UDCA conversion rate of each enzyme at different 7-Keto-LCA concentrations according to pH adjustment. (○: ABP-5 with pH control ■: ABP-5 W/O pH control)

### Mode of Disclosure

Hereinafter, the present disclosure will be described in more detail through examples. These examples are intended solely to illustrate the disclosure, and it will be apparent to one of ordinary skill in the art that the scope is not to be construed as limited by these examples.

### [Example]

### <Example 1: Development of ABP-5 expression vector and expression strain

From among the amino acid sequence of *R. gnavus-derived* 7-beta-HSDH, the region around 200 to 215 is involved in substrate binding, and the region around 240 to 259 is involved in stereoselectivity of the substrate. In the case where, from among amino acids involved in binding, methionine at position 207, threonine at position 210, and glutamine at position 212 are replaced, and from among amino acids involved in substrate stereoselectivity,glutamic acid at position 252 is replaced, the activity was maintained even when the substrate concentration was increased.

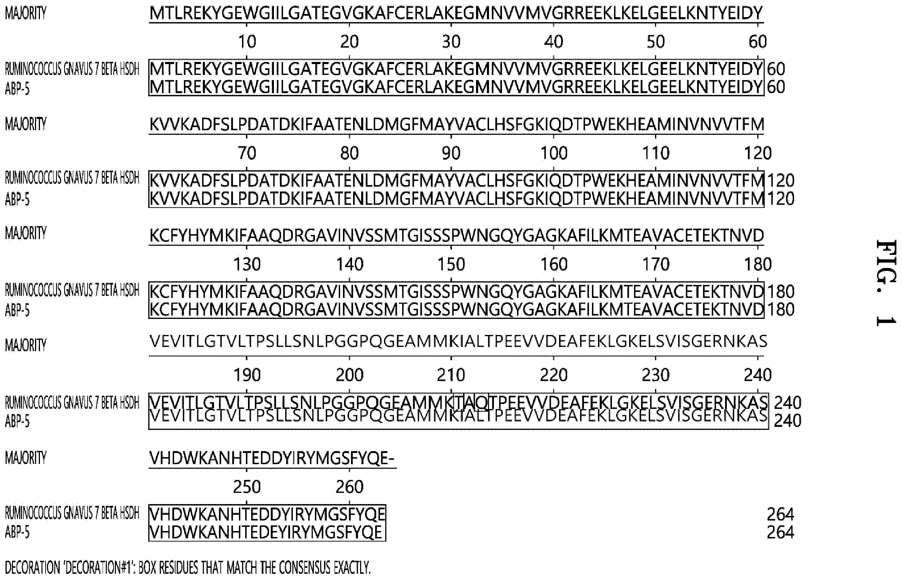

Therefore, ABP-5, a variant of 7-beta-HSDH, contains M207V, T210I, Q212L, and D252E mutations in the existing amino acid of 7-beta-HSDH. For example, ABP-5 is a variant in which methionine, the 207th amino acid, is replaced with valine, threonine, the 210th amino acid, is replaced with isoleucine, glutamine, the 212th amino acid, is replaced with leucine, and aspartic acid, the 252nd amino acid, is replaced with glutamic acid (FIG. 1).

To insert the gene of recombinant enzyme ABP-5, an improved 7-beta-HSDH enzyme derived from *Ruminococcus gnarvus* strain, into *Escherichia coli.* (*E. coli*) MG1655 strain, codon optimization was performed using the amino acid sequence of ABP-5 using the codon optimization tool provided on the website of Integrated DNA Technologies, Inc. and then commissioned Macrogen (Seoul, Republic of Korea) for DNA synthesis.

Specifically, to obtain the codon-optimized ABP-5 gene (SEQ ID NO: 2) for this experiment and the *R. gnavus* 7-beta-HSDH (SEQ ID NO: 4) for comparison, gene amplification was performed by PCR using primers ABP-5_F (SEQ ID NO: 5), ABP-5_R (SEQ ID NO: 6), 7-beta-HSDH-F (SEQ ID NO: 7), and 7-beta-HSDH-R (SEQ ID NO: 8).

To construct a vector to be used for expression, pCDFDuet-1, an expression vector for *E. coli,* was used. In order to replace the T7 promoter present in the vector with the Tac promoter, a method using hangover PCR was used, and the primers F_Tac_overhang and R_Tac_overhang used at this time were constructed as the sequences shown in SEQ ID NOs: 2 and 3. To prepare the vector, the whole vector was subjected to a reverse PCR reaction using the DNA polymerase (Q5^{®} High-Fidelity DNA Polymrase, NEB) under conditions: 98 °C for 10 seconds, 60 °C for 30 seconds, and 72 °C for 30 seconds per 1 kb of gene size, and 30 cycles of this cycle (98 °C for 10 seconds, 50 °C for 30 seconds, and 72 °C for 30 seconds per 1 kb of gene size), and finally, 72 °C for 5 minutes. Then, to remove pCDFDuet-1, which is the template vector, the treatment using Dpnl (NEB) was performed for 1 hour at 37 °C, and the resultant vector was then inserted into the cloning host, *E. coli* DH5a, and then the constructed vector was amplified and used.

In addition, to replace the ampicillin cassette of the pCDFduet-1 vector with the kanamycin cassette, all vector-F and R primers (SEQ ID NOs: 9 and 10) were used and DNA polymerase (Q5^{®} high-fidelity DNA polymerase, NEB) was used. The whole vector was subjected to reverse PCR reaction under the following conditions: 98 °C for 10 seconds, 55 °C for 30 seconds, and 72 °C for 30 seconds of reaction per 1 kb of gene size, and 30 cycles of this cycle (98 °C for 10 seconds, 50 °C for 30 seconds, and 72 °C for 30 seconds of reaction per 1 kb of gene size), and finally 72 °C for 5 minutes of reaction. To remove the remaining template vector, Dpnl (NEB) was performed at 37 °C for 1 hour. The kanamycin cassette was reacted using kanamycin-F,R primers ( SEQ ID NOs: 11 and 12) using GXL DNA polymerase (Takarasa, Japan) for 30 seconds at 98 °C, 10 seconds at 98 °C, 50 °C for 30 seconds, and 72 °C for 30 seconds per 1 kb of gene size, and this cycle (98 °C for 10 seconds, 50 °C for 30 seconds, and 72 °C for 30 seconds per 1 kb of gene size) was performed 30 times, and finally the reaction was performed at 72 °C for 5 minutes. Reverse PCR-reacted vector and kanamycin cassette were cloned by in-fusion method using In Fusion HD Enzyme (Takara, Japan), and the finally constructed expression vector was named pCDFDuet-1-Tac-Kan vector.

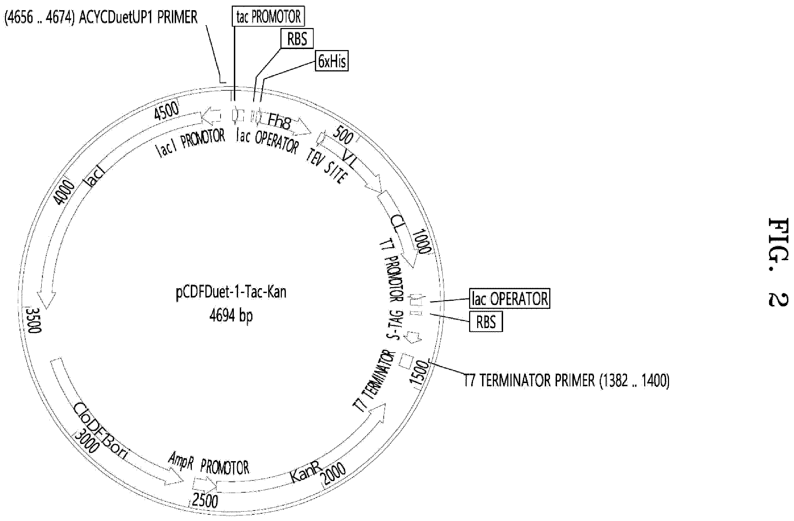

Then, the pCDFDuet-1-Tac-Kan vector (FIG. 2) was treated with Ncol and BamHl restriction enzymes, and then the amplified ABP-5 enzyme and *R. gnavus* 7-beta-HSDH gene were inserted by in-fusion method using In Fusion HD Enzyme (Takara, Japan), and finally the recombinant vector was inserted into *E. coli* MG1655 strain.

To screen pCDFDuet-1-Tac-Kan inserted strains containing the ABP-5 gene after transformation, the strains were seeded on LB medium containing kanamycin at a concentration of 50 µg/mL and cultured at 37 °C for 1 day. Then, colony PCR was performed using BioFACT's 2X Taq PCR pre-Mix product and ABP-5_F and ABP-5_R primers or 7-beta-HSDH-F and 7-beta-HSDH-R primers, and finally strains, into which pCDFDuet-1-Tac-Kan containing ABP-5 gene and *R. gnavus* 7-beta-HSDH gene were inserted, was screened. *E. coli* MG1655 carrying the pCDFDuet-1-Tac-Kan vector containing the ABP-5 gene was named *'E. coli* ABP-5' and was deposited at the Korea Institute of Biotechnology on September 17, 2021 and assigned Accession No. KCTC 14715 BP. In addition, the improved 7-beta-HSDH expressed in the strain was named ABP-5.

**[Table 1]**

| SEQ ID NO | Sequence name | Sequence (nucleic acid sequence 5'→ 3'/ amino acid sequence N-terminal → C-terminal) |
|---|---|---|
| 1 | ABP-5 amino acid sequence | SEQ ID NO: 1 |
| 2 | ABP-5 sequence | SEQ ID NO: 2 |
| 3 | *R. gnavus* 7-beta-HSDH amino acid sequence | SEQ ID NO: 3 |
| 4 | *R. gnavus* 7-beta-HSDH sequence | SEQ ID NO: 4 |
| 5 | ABP-5_F | SEQ ID NO: 5 |
| 6 | ABP-5_R | SEQ ID NO: 6 |
| 7 | 7-beta-HSDH-F | SEQ ID NO: 7 |
| 8 | 7-beta-HSDH-R | SEQ ID NO: 8 |
| 9 | F_Tac_overhang | SEQ ID NO: 9 |
| 10 | R_Tac_overhang | SEQ ID NO: 10 |
| 11 | Kanamycin-F | SEQ ID NO: 11 |
| 12 | Kanamycin-R | SEQ ID NO: 12 |
| 13 | All vector-F | SEQ ID NO: 13 |
| 14 | All vector-R | SEQ ID NO: 14 |

### <Example 2: Confirmation of ABP-5 enzyme expression and UDCA conversion rate of 7-keto LCA>

Confirmed were: the expression of the recombinant protein using recombinant *E. coli* ABP-5 (Accession No. KCTC 14715 BP) prepared in Example 1 above, which was transformed to overexpress the gene for overexpression of the ABP-5 enzyme; and the conversion of 7-Keto-LCA to UDCA using the recombinant protein.

As for the growth of the strain (*E*. *coli* ABP-5 (Accession No. KCTC 14715 BP)), OD values at a wavelength of 600 nm was measured using a UV-visible spectrophotometer (UV-1800, SHIMADZU). After culturing, protein analysis was performed using the sodium dodecyl sulfate polyacrylamide gel electrophoresis (Mini-PROTEIN Tetra system, Bio-Rad), and analysis was performed using polyacrylamide gel for analysis (Mini-PROTEAN TGX Precast Gels) and sample buffer (2x Laemmli Sample Buffer).

UFLC (LC-20AD, SHIMADZU) was used to confirm the conversion of 7-Keto-LCA to UDCA using recombinant protein ABP-5; andHPLC Chromaster (Hitachi, Japan) and ACCUCORE C30 2.6UM 250X2.1 MM COLUMN were used to analyze 3-HP in the culture medium, and the refractive index detector (RID) was used. As a solvent, 2.2 mL of 1 M K₂HPO₄ (pH 3.0), 370 mL of HPLC water, 300 mL of acetonitrile, and 400 mL of methanol were mixed, followed by filtering using a 0.2 µm pore size filter. Then, the analysis was performed under the condition of using 0.4 mL of solvent per minute at 40 °C.

### 2-1. Recombinant protein (ABP-5) expression and SDS-PAGE confirmation

For pre-culture of the strain (*E*. *coli* ABP-5), Luria bertani (LB) medium was used, supplemented with 50 µg/L kanamycin, 0.5 g/L glucose, 5 g/L glycerol, and 2 g/L lactose. The strain used for seeding was a strain stored by the glycerol stock method at -80 °C, and was seeded at a concentration of 1% in LB medium and pre-cultured at 180 rpm and 37 °C. In addition, after pre-culture, it was collected in an exponential phase, washed twice with sterile distilled water, and used for main culture.

Meanwhile, 50 mL of LB_Kan medium was used in a 250 mL baffled flask for the production of the ABP-5 enzyme and the control, *R. gnavus* 7-beta-HSDH. The culture was carried out using a baffled flask, stirring speed was 180 rpm, and the culture of E. *coli* and expression of enzymes were carried out at 37 °C.

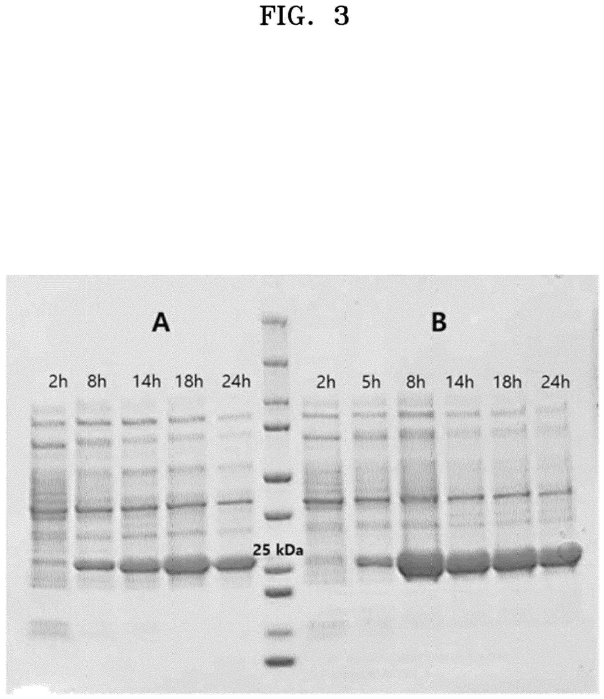

The cultures for each culture time were collected and centrifuged to remove the supernatant, and then,the recovered *E. coli were* washed twice with sterile distilled water, resuspended in 50 mM phosphate buffer (pH 8.0), and crushed using an ultrasonic disruptor, and the expression of proteins was confirmed (FIG. 3). As a result, it was confirmed that the expression of the ABP-5 enzyme and the control group *R. gnavus* 7-beta-HSDH proceeded smoothly after 2 hours.

### 2-2. Measurement of R. gnavus-derived 7-beta-HSDH and ABP-5 activities

To measure the activity of ABP-5 enzyme, *R. gnavus-*derived 7-beta-HSDH and ABP-5 enzymes were expressed and used to measure enzymatic activity using the conversion of 7-Keto-LCA into UDCA.

In the case of enzymatic conversion of 7-Keto-LCA to UDCA, iNADPH is required as a coenzyme. Therefore, to measure the enzyme activity, the absorbance of the 340 nm wavelength range of NADPH was used, and measured using an ultraviolet (UV)-visible spectrophotometer (UV-1800, SHIMADZU).

For the enzyme reaction, NADPH was added at a concentration of 0.5 mM, and 50 mM phosphate buffer (pH 8.0) was used as the reaction buffer. The substrate 7-Keto-LCA was added at a concentration of 10 g/L, and for the *R. gnavus* 7-beta-HSDH and ABP-5 enzymes, the cell lysates thereof were added in the amount of 2 µL, and the reaction was cause to occur to a final volume of 2 mL.

In the case of activity measurement, after adding each enzyme, the change in absorbance at 340 nm was measured at 1-minute intervals, and the measured absorbance change was plotted against the NADPH-standard curve (340 nm, 0.01 - 0.1 mM NADPH) to determine the activity of the enzyme. The Bradford protein quantification method was used to measure the protein concentration of the cell lysates, and each measured protein concentration was calculated.

The enzymatic activities of *R. gnavus*-derived 7-beta-HSDH and ABP-5 were compared by using *R. gnavus*-derived 7-beta-HSDH andrecombinant *E. coli* ABP-5 into which an expression vector having the ABP-5 enzyme genes inserted thereinto, and E. *coli* R. gnavus-derived 7-beta-HSDH, and10 colonies obtained at the time of transformation were selected and expressed by the expression method of Example 2.1, and the activities of *R. gnavus*-derived 7-beta-HSDH and ABP-5, recombinant 7-beta-HSDH expressed in recombinant *E. coli,* were measured (Table 2). The results showed an average activity of 41.62±4.55 U/mg for *R. gnavus*-derived 7-beta-HSDH and 49.13±4.72 U/mg for ABP-5, indicating an increase in activity of about 18.10% compared to the existing enzyme.

**[Table 2]**

| Colony no. | Activity (U/mg) | |
|---|---|---|
| | *R. gnavus* 7-beta-HSDH | ABP-5 |
| 1 | 39.35 | 45.33 |
| 2 | 38.24 | 46.37 |
| 3 | 42.74 | 48.50 |
| 4 | 45.87 | 51.64 |
| 5 | 41.12 | 53.73 |
| 6 | 33.98 | 55.42 |
| 7 | 35.45 | 39.82 |
| 8 | 47.90 | 48.94 |
| 9 | 45.15 | 53.84 |
| 10 | 43.32 | 47.70 |
| Average | 41.62±4.55 | 49.13±4.72 |

### 2-3. Conversion of 7-Keto-LCA into UDCA using ABP-5 enzyme and R. gnavus-derived 7-beta-HSDH

Conversion of 7-Keto-LCA to UDCA was performed using the expressed ABP-5 enzyme and the control *R. gnavus* 7-beta-HSDH.

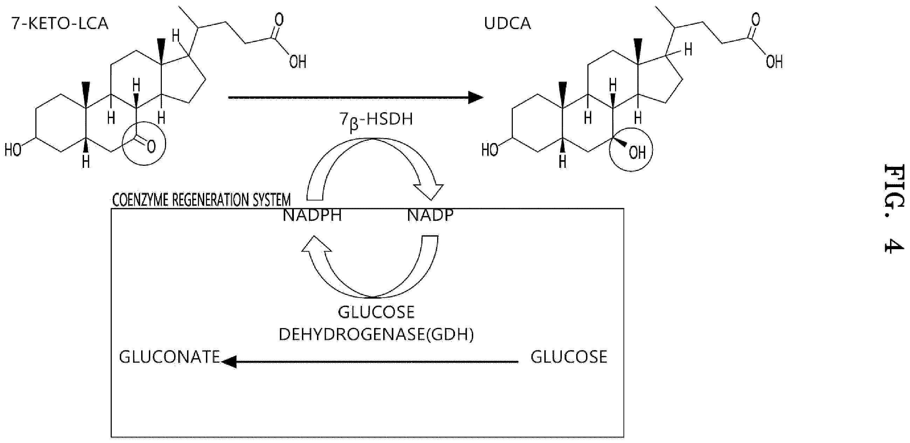

7-beta-HSDH, which is an enzyme used to convert 7-Keto-LCA to UDCA, requires NADPH as a coenzyme. However, since this reaction is an equivalent-to-equivalent reaction, the reaction requires the same amount of NADPH as the 7-Keto-LCA to be converted. This can lead to an inefficient reaction that requires a large amount of NADPH, and to solve this problem, a reaction system including a co-factor regeneration system as shown in FIG. 4 was constructed and the conversion was performed.

For the conversion reaction, 50 mM phosphate buffer (pH 8.0) was used, 7-Keto-LCA (10, 20, 30, 40, or 50 g/L), 1 mM NADPH, 200 mM glucose, 2 U/mL of glucose dehydrogenase (GDH ), and 4 U/mL of each of ABP-5 enzyme or *R. gnavus* 7-beta-HSDH were used for the reaction at 30 °C and at the rate of 150 rpm.

As a result of the reaction, it was confirmed that for both enzymes, the conversion rate into UDCA was 100% up to a concentration of 20 g/L of 7-Keto-LCA. However, in the case of conversion using *R. gnavus* 7-beta-HSDH, the conversion rate decreased to 55.12% when the concentration of 7-Keto-LCA was 30 g/L, 23.53% when the concentration was 40 g/L, and 5.37% when the concentration was 50 g/L, confirming that the conversion rate decreased with the increase of the concentration of the substrate.

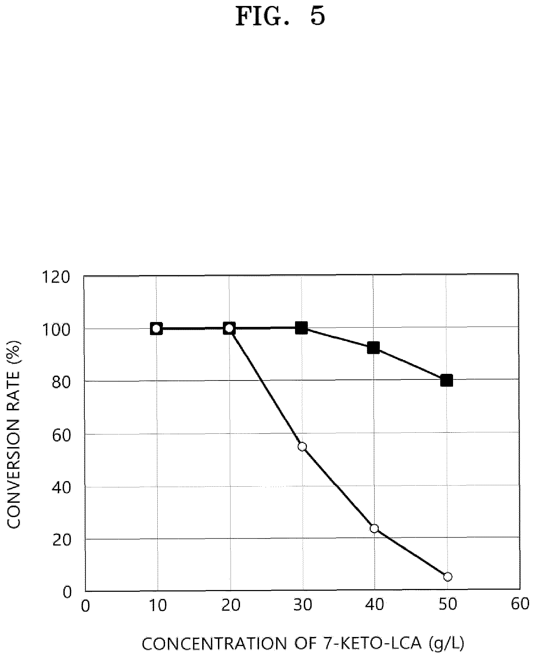

On the other hand, when the conversion was performed using ABP-5 enzyme, the conversion rate decreased to 100% when the concentration of 7-Keto-LCA was 30 g/L, 92.44% when the concentration was 40 g/L, and 79.82 % when the concentration was 50 g/L, confirming that the decrease in conversion rate with increasing substrate concentration was significantly lower than that of *R. gnavus* 7-beta-HSDH (FIG. 5). In other words, the improved enzyme variant showed the same conversion efficiency at 30 g/L as well as at 20 g/L, the concentration of 7-Keto-LCA at which the existing conversion activity was maintained, and confirmed that conversion to UDCA was possible at 40 g/L and 50 g/L concentrations of 7-Keto-LCA.

Based on these results, it was found that ABP-5, which is the 7-beta-HSDH enzyme variant, is resistant to inhibition by increasing substrate concentrations compared to the conventional enzyme, and this advantage can be utilized for various process benefits.

### <Example 3: Conversion of 7-keto LCA to UDCA using ABP-5 enzyme under pH control>

As shown in Example 2, it was confirmed that the inhibition of conversion to UDCA occurring at concentrations of 7-Keto-LCA, which is equal to or greater than 40 g/L, was somewhat alleviated when the newly developed enzyme ABP-5 was used, unlike *when R. gnavus* 7-beta-HSDH was used. However, when the concentration of 7-Keto-LCA was increased to equal to or greater than 50 g/L, the conversion rate tended to decrease significantly to 79.82 %.

In the case of GDH, which is used as a coenzyme regeneration system for the conversion of 7-Keto-LCA to UDCA, glucose is converted to gluconic acid and NADP is used as a coenzyme. Therefore, as the reaction proceeds, the pH of the reaction solution decreases due to the accumulated gluconic acid, and when the reaction starts at a starting pH of 8.0, the pH tends to decrease to 7.2 at the end. This decrease in pH was thought to be the cause of the inhibition of activity, so the pH of the reaction solution was measured every hour and the pH was readjusted to be 8.0 to confirm the ability to conversion.

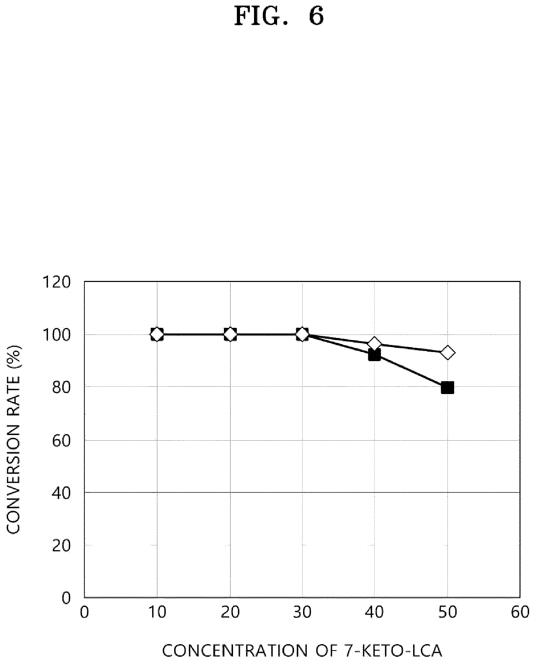

For the conversion reaction, 50 mM phosphate buffer (pH 8.0) was used, 7-Keto-LCA (10, 20, 30, 40, or 50 g/L), 1 mM NADPH, 200 mM glucose, 2 U/mL of glucose dehydrogenase (GDH ), and 4 U/mL of each of ABP-5 enzyme or *R. gnavus* 7-beta-HSDH were used for the reaction at 30 °C and at the rate of 150 rpm. In addition, the pH of the reaction solution was adjusted to be 8.0 with 5 N NaOH after each hourly measurement using a pH meter (FiveEasy, Mettler toledo) to perform the conversion. As a result, it was confirmed that the conversion rate was improved by 4.2% (92.44 → 96.24%) at 40 g/L and by 12.43% (79.82% → 92.45%) at 50 g/L, compared to the condition where the pH was not adjusted (FIG. 6).

The aforementioned description is for illustrative purposes only, and one having ordinary skill in the art to which the present disclosure belongs will understand that it could easily be adapted to other specific forms without changing the technical concept or essential features. Therefore, the embodiments described above should be understood as illustrative in all respects and not limiting.

BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: Ace biopharma CO., LTD
Ace biopharma CO., LTD
41, Dongsan-ro 8-gil, Seocho-gu, Seoul
Republic of Korea

I. IDENTIFICATION OF THE MICROORGANISM
Identification reference given by the
DEPOSITOR:

Escherichia coli ABP-5 | Accession number given by the
INTERNATIONAL DEPOSITARY AUTHORITY:

KCTC 14715BP
II. SCIENTIFIC DESCRIPTION AND/OR PROPOSED TAXONOMIC DESIGNATION
The microorganism identified under I above was accompanied by:
[√ ] a scientific description
[ ] a proposed taxonomic designation
(Mark with a cross where applicable)
III. RECEIPT AND ACCEPTANCE
This International Depositary Authority accepts the microorganism identified under I above, which was received by it
on September 17, 2021.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeolllabuk-do 56212
Republic of Korea | Signature(s) of person(s) having the power to represent the
International Depositary Authority or of authorized
official(s):

Song-Gun Kim

KIM, Song-Gun, Director
Date: September 17, 2021

Form BP/4 (KCTC Form 17)
sole page

## Claims

1. A 7 beta-hydroxysteroid dehydrogenase (7-beta-HSDH) variant comprising an amino acid sequence mutation of *RUMINOCOCCUS GNAVUS* strain-derived 7-beta-HSDH, wherein
the amino acid sequence mutation comprises any one or more mutations selected from the group consisting of:
1) M207X₁,
2) T210X₂ and
3) Q212X₃, wherein
X₁ represents an amino acid other than methionine (M),
X₂ represents an amino acid other than threonine (T), and
X₃ represents an amino acid other than glutamine (Q).

2. The 7-beta-HSDH variant of claim 1, wherein the 7-beta-HSDH additionally has at least one mutation in a sequence motif corresponding to an amino acid sequence having at least 80% sequence homology to SEQ ID NO: 1.

3. The 7-beta-HSDH variant of claim 2, wherein a sequence motif 207 to 212 of SEQ ID NO: 1 is VMKIAL.

4. The 7-beta-HSDH variant of claim 1, wherein the *RUMINOCOCCUS GNAVUS* strain-derived 7-beta-HSDH recognizes 7-keto lithocholic acid (7-Keto-LCA) as a substrate and induces catalysis of stereospecific enzymatic reduction to a 7th carbon of 7-Keto-LCA.

5. The 7-beta-HSDH variant of claim 1, wherein the amino acid sequence mutation
4) additionally has D252X₄ wherein X₄ represents an amino acid other than aspartic acid (D).

6. A recombinant vector comprising the 7-beta-HSDH variant of any one of claims 1 to 5.

7. The recombinant vector of claim 6, wherein the 7-beta-HSDH variant consists of a nucleotide sequence encoding the amino acid sequence of SEQ ID NO: 1.

8. The recombinant vector of claim 6, wherein the 7-beta-HSDH variant consists of the nucleotide sequence of SEQ ID NO: 2.

9. The recombinant vector of claim 6, wherein the recombinant vector comprises a Tac promoter and a kanamycin resistance gene.

10. A cell transformed with the recombinant vector of claim 6.

11. The cell of claim 10, wherein the cell is transformed by introducing the recombinant vector into *Escherichia coli.*

12. The cell of claim 11, which is deposited under Accession No. KCTC 14715 BP.

13. A method of producing ursodeoxycholic acid (UDCA) for increasing the conversion rate of 7-keto lithocholic acid (7-Keto-LCA) to UDCA, the method comprising converting 7-Keto-LCA to UDCA by 7 beta-hydroxy steroid dehydrogenase (7-beta-HSDH) of which the substrate binding site is mutated.

14. The method of claim 13, wherein the substrate binding site is a 200 to 215 sequence motif of an amino acid sequence of 7-beta-HSDH.

15. The method of claim 13, wherein the mutation comprises one or more mutations selected from the group consisting of
1) M207X₁,
2) T210X₂ and
3) Q212X₃
, wherein
X₁ represents an amino acid other than methionine (M),
X₂ represents an amino acid other than threonine (T), and
X₃ represents an amino acid other than glutamine (Q).

16. A method of increasing the conversion rate of 7-keto lithocholic acid (7-Keto-LCA) to ursodeoxycholic acid (UDCA), the method comprising converting 7-Keto-LCA to UDCA by 7 beta-hydroxy steroid dehydrogenase (7-beta-HSDH) of which the substrate binding site is mutated.

17. The method of claim16, wherein the substrate binding site is 200 to 215 sequence motif of an amino acid sequence of 7-beta-HSDH.

18. The method of claim 16, wherein the mutation comprises one or more mutations selected from the group consisting of
1) M207X₁,
2) T210X₂ and
3) Q212X₃, wherein
X₁ represents an amino acid other than methionine (M),
X₂ represents an amino acid other than threonine (T), and
X₃ represents an amino acid other than glutamine (Q).
